# EUROPÄISCHE PATENTSCHRIFT

(11) **EP 2 714 645 B1**
(45) Veröffentlichungstag und Bekanntmachung des Hinweises auf die Patenterteilung: **05.08.2015**
(21) Anmeldenummer: 12721845.1
(22) Anmeldetag: 16.05.2012
(51) Int. Cl.: C07C 227/18, C07C 229/16

(54) **VERFAHREN ZUR HERSTELLUNG VON AMINOPOLYCARBOXYLATEN**
METHOD FOR PRODUCING AMINOPOLYCARBOXYLATES
PROCÉDÉ DE PRODUCTION D'AMINOPOLYCARBOXYLATES

(30) Priorität: 23.05.2011 EP 11167136
(43) Veröffentlichungstag der Anmeldung: 09.04.2014
(73) Patentinhaber: BASF SE, 67056 Ludwigshafen (DE)
(72) Erfinder: BAUMANN, Robert, 68529 Mannheim (DE); BIEL, Markus Christian, 68159 Mannheim (DE); FRANZKE, Axel, 68161 Mannheim (DE); OFTRING, Alfred, 67098 Bad Dürkheim (DE); TEICH, Friedhelm, Neckarhausen 68535 (DE); KLINGELHOEFER, Paul, 68165 Mannheim (DE); SCHRÖTER, Marie Katrin, 67146 Deidesheim (DE)
(86) Internationale Anmeldenummer: PCT/EP2012/059126
(87) Internationale Veröffentlichungsnummer: WO 2012/159952

(56) Entgegenhaltungen:
- EP-A1- 0 506 973
- WO-A1-00/66539
- WO-A1-98/50150
- WO-A1-03/051513
- WO-A1-2011/113822
- DE-A1- 3 505 208
- US-A1- 2011 257 431
- DATABASE CAPLUS [Online] CHEMICAL ABSTRACTS SERVICE, COLUMBUS, OHIO, US; XP002682867, Database accession no. 1999:378457 -& JP 11 158130 A (KAO) 15. Juni 1999 (1999-06-15)

## Beschreibung

Die Erfindung betrifft ein Verfahren zur Herstellung von Aminopolycarboxylaten ausgehend von den entsprechenden Polyalkanolaminen durch katalytische oxidative Dehydrierung unter Einsatz einer Base.

Die oxidative Dehydrierung von Aminoalkoholen mit Alkalihydroxiden wird üblicherweise im wässrigen Medium unter Druck und bei Temperaturen von 140 bis 220 °C unter Verwendung von Kupfer enthaltenden Katalysatoren durchgeführt. Die Katalysatoren bestehen z. B. aus undotiertem oder dotiertem Raney-Kupfer (z. B. WO 00/066539). Als Dotierstoffe werden in der Regel ein oder mehrere Metalle, z. B. Pt, Fe, Cr, Mo, V, Bi, Sn, Sb, Pb, Ge oder Ag verwendet.

In anderen Fällen wird Kupfer direkt oder über Ankermetalle (z. B. Os, Ir, Rh, Pt oder Pd) auf alkalistabile Träger aufgebracht (z. B. in WO 98/50150). Auch Kupferfällkatalysatoren mit weiteren Metalloxiden wurden beschrieben (z. B. in WO 03/051513 (Cu, Fe) oder in EP 0 506 973 (Cu, Zr, Ca)). Vereinzelt ist über die Umsetzung an Edelmetallsystemen berichtet worden (z. B. in EP 0 201 957).

Ein Problem bei der Herstellung insbesondere von komplexbildenden Aminopolycarboxylaten wie Methylglycindiessigsäure, Glutaminsäurediessigsäure, Nitrilotriessigsäure, Ethylendiamintetraessigsäure und deren Salzen aus den entsprechenden Polyalkanolaminen besteht darin, dass bei einer dem Stand der Technik entsprechenden Durchführung Nebenprodukte mit geringerer Wirksamkeit entstehen. Hierzu gehören insbesondere Verbindungen, die aus C-N bzw. C-C-Bindungsbrüchen hervorgehen. Am Beispiel des Aminopolycarboxylates Methylglycindiessigsäure-Trinatriumsalz (MGDA-Na₃) sind dies zum Beispiel das Carboxymethylalanin-Dinatriumsalz (C-N-Bindungsspaltung) und das N-Methyl-N-Carboxymethylalanin (C-C-Bindungsspaltung).

Es war daher Aufgabe der Erfindung, ein technisch einfaches Verfahren zur Herstellung von Aminopolycarboxylaten ausgehend von den entsprechenden Polyalkanolaminen durch katalytische oxidative Dehydrierung zur Verfügung zu stellen, wonach ein Produkt erhalten wird, das unmittelbar und ohne aufwändige weitere Aufreinigung einen hohen Reinheitsgrad aufweist. Dies ist gleichbedeutend mit einer hohen Ausbeute von mindestens 85 Mol-% zum gewünschten Aminopolycarboxylat oder anders ausgedrückt, die Nebenprodukte sollen nicht mehr als 15 Gew.-% in Bezug auf das gewünschte Produkt ausmachen.

Gegebenenfalls können vor der entsprechenden Anwendung noch einfache Nachbehandlungsmaßnahmen vorgenommen werden: im Falle einer Suspensionsfahrweise kann der Katalysator sedimentiert und/oder abfiltriert werden. Außerdem kann anschließend noch ein gewünschter Wassergehalt eingestellt und/oder eine Bleichung z. B. mit Wasserstoffperoxid oder UV-Licht durchgeführt werden.

Neben den Salzen (Aminopolycarboxylaten) selbst sind nach Ansäuern auch die entsprechenden Aminopolycarbonsäuren zugänglich.

Gleichzeitig sollen die Reaktionsbedingungen der oxidativen Dehydrierung eine möglichst lange Recyclierbarkeit des verwendeten Katalysators gewährleisten.

Diese Aufgabe wurde gelöst durch ein Verfahren zur Herstellung von Aminopolycarboxylaten ausgehend von den entsprechenden Polyalkanolaminen durch oxidative Dehydrierung in Gegenwart eines Katalysators enthaltend 1 bis 90 Gew.-% Kupfer, bezogen auf das Gesamtgewicht des Katalysators, unter Einsatz einer Base, das dadurch gekennzeichnet ist, dass zunächst eine Teilumsetzung des Polyalkanolamins zu einem das Aminopolycarboxylat enthaltenden Reaktionsgemisch bei einer Temperatur im Bereich von 140 bis 180 °C durchgeführt wird, bis mindestens 10 bis 90 Mol-% des Polyalkanolamins abreagiert sind, und dass anschließend die Umsetzung bei erhöhter Temperatur fortgeführt wird.

Die katalytische oxidative Dehydrierung von Polyalkanolaminen kann durch die nachfolgende Reaktionsgleichung dargestellt werden: 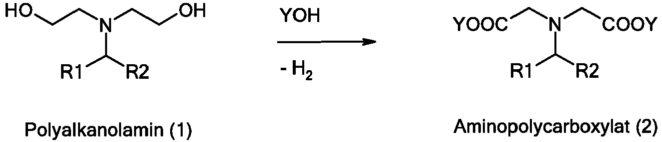
R1 = H, -COOX mit X = Alkali-, Erdalkalimetall oder Wasserstoff
R2 = Alkyl-, Alkenyl-, Alkinyl-, Aralkyl-, Hydroxyalkyl-, Hydroxyaralkyl-, Alkylencarboxyl-, Alkylensulfonat- oder Bis(hydroxyethyl)aminoalkylen-Rest
Y = Alkali- oder Erdalkalimetall

Diese Reaktion kann mit Raney-Kupfer als Katalysator in guten Ausbeuten und guter Selektivität durchgeführt werden. Allerdings lässt sich aufbauend auf diesem Katalysatorsystem kein wirtschaftliches Verfahren betreiben, da Raney-Kupfer bei hohen Temperaturen und unter basischen Bedingungen sehr schnell deaktiviert und so nur in sehr begrenztem Umfang recycelt werden kann. Aus diesem Grund sind in einem geeigneten Katalysator neben Kupfer auch noch weitere Komponenten in seiner Aktivmasse enthalten, die eine höhere Standzeit und Stabilität gewährleisten, gleichzeitig aber auch die Selektivität der Dehydrierung im Vergleich zum Raney-Kupfer verringern können. So liefert zum Beispiel eine dem Stand der Technik entsprechende Ausführung mit einem Cu/ZrO₂-Katalysator zwar einen vollständigen Umsatz des Polyalkanolamins (1) ALDE-Na (R1 = COONa, R2 = CH₃) und zeigt damit in Bezug auf die oxidative Dehydrierung eine hohe katalytische Aktivität, führt jedoch lediglich zu einer MGDA-Na₃ (Methylglycindiessigsäure-Trinatriumsalz) Ausbeute von 72.5%. Hauptnebenprodukt ist mit einer Ausbeute von 25.6% das aus einer C-N-Bindungsspaltung hervorgehende Carboxymethylalanin-dinatriumsalz, im Folgenden als CMA-Na₂ bezeichnet. Die Bildung derartiger Abbauprodukte bei der oxidativen Dehydrierung ist literaturbekannt.

Demgegenüber haben die Erfinder gefunden, dass die Selektivität der obigen Dehydrierung durch Auswahl spezifischer Reaktionsbedingungen deutlich gesteigert werden konnte.:
In Bezug auf die Reaktionstemperatur wurde am Beispiel des Polyalkanolamins (1) mit R1 = COONa und R2 = CH₃ (im Folgenden als ALDE-Na₃ bezeichnet) gefunden, dass sich bei Durchführung der Reaktion bei konstant 170 °C weniger CMA-Na₂ als Nebenprodukt (C-N-Bindungsbruch) bildet als bei konstant 190 °C, gleichzeitig aber die zum Vollumsatz notwendige Reaktionszeit deutlich zunimmt. Dies ist unter wirtschaftlichen Gesichtspunkten nachteilig, da dieses Vorgehen mit deutlich geringeren Raum-Zeit-Ausbeuten verbunden ist. Überraschenderweise wurde jedoch gefunden, dass die CMA-Na₂-Bildung im überwiegenden Maße aus dem eingesetzten Polyalkanolamin selbst heraus erfolgt, und nicht aus Intermediaten der Reaktion. Aus diesem Grund ist es zur Steigerung der Selektivität ausreichend, die Reaktion bei niedrigerer Temperatur zu starten und je nach gewünschter Selektivität die Reaktionstemperatur nach Umwandlung des hierzu notwendigen Anteils an Edukt in Intermediat bzw. Produkt direkt oder schrittweise zu erhöhen und so Reaktionsgeschwindigkeit bei praktisch gleichbleibender Selektivität zu gewinnen. Auf diese Weise kann im Vergleich zur Reaktion bei konstant niedriger Temperatur das Aminopolycarboxylat in praktisch gleicher Selektivität jedoch deutlich kürzerer Reaktionszeit erhalten werden.

Die katalytische oxidative Dehydrierung des Polyalkanolamins wird somit erfindungsgemäß in der Weise durchgeführt, dass zunächst eine Teilumsetzung des Polyalkanolamins bei einer niedrigeren Temperatur, im Bereich von 140 bis 180 °C durchgeführt wird, bis mindestens 10 bis 90 Mol-% des Polyalkanolamins abreagiert sind, und dass anschließend die Temperatur erhöht wird.

Bevorzugt beträgt der Temperaturbereich, in dem die Teilumsetzung durchgeführt wird, 150 bis 175 °C, insbesondere 165 bis 175 °C.

Vorteilhaft wird die Teilumsetzung bei niedrigerer Reaktionstemperatur durchgeführt, bis die Menge des abreagierten Polyalkanolamins 30 bis 90 Mol-%, bevorzugt 50 bis 80 Mol-% beträgt.

Nach der Teilumsetzung wird die Temperatur bevorzugt direkt, d.h. in einem einzigen Schritt, oder schrittweise, d.h. in mehreren Schritten, auf 180 bis 200 °C, insbesondere auf 185 bis 195 °C, erhöht.

Bevorzugt wird die katalytische oxidative Dehydrierung des Polyalkanolamins in Gegenwart von Wasser als Lösungsmittel durchgeführt.

Es wurde gefunden, dass neben dem oben beschriebenen Einfluss der Temperatur auf die Selektivität der Reaktion auch die Konzentration des Wassers im Reaktionsgemisch eine Rolle spielt:
Das bevorzugt als Lösungsmittel eingesetzte Wasser übt bei zunehmender Konzentration überraschenderweise nicht nur einen verdünnenden Effekt aus, der die Reaktionsgeschwindigkeit erniedrigt, sondern hat auch einen negativen Einfluss auf die Selektivität der Reaktion. So wurde gefunden, dass die CMA-Na₂-Ausbeute bei Einsatz des Eduktes ALDE-Na in einer Lösung mit 58 Gew.-% Wasser bereits 12% beträgt, bei lediglich 50% MGDA-Na₃-Ausbeute, während in einer konzentrierteren Fahrweise (37 Gew.-% statt 58 Gew.-% Wasser im Reaktionsgemisch) eine ähnliche CMA-Na₂-Ausbeute bei einer MGDA-Na₃-Ausbeute von immerhin 81% erreicht wird. Noch deutlich geringere Wassergehalte in der Reaktionsmischung sind in der Praxis schlechter realisierbar, da aufgrund des viskosen Verhaltens der Edukte und Produkte, der Katalysator nicht mehr optimal suspendiert werden kann.
Das Verfahren wird daher bevorzugt in der Weise durchgeführt, dass die Konzentration des Wassers im Reaktionsgemisch 30 bis 80 Gew.-%, bevorzugt 35 bis 60 Gew.-%, insbesondere 40 bis 55 Gew.-%, bezogen auf das Gesamtgewicht des Reaktionsgemisches, beträgt.
Darüber hinaus wurde gefunden, dass mit steigender Katalysatorkonzentration in der Reaktionsmischung nicht nur die Reaktionsgeschwindigkeit der oxidativen Dehydrierung, sondern überraschenderweise auch die Selektivität der Reaktion zunimmt, so dass zum Beispiel durch Erhöhung der Katalysatormenge von 3 g/mol ALDE-Na (R1 = COONa, R2 = CH₃) auf 40 g/mol ALDE-Na die MGDA-Na₃-Ausbeute bei gleichbleibendem Umsatz von 72.5% auf knapp 90% steigt und die Ausbeute an CMA-Na₂ im Gegenzug sinkt.

Erfindungsgemäß wird somit das Verfahren bevorzugt in der Weise durchgeführt, dass die eingesetzte Katalysatormenge so bemessen wird, dass sie nur 0,40 g bis 2,00 g Kupfer, bevorzugt 1,00 g bis 1,70 g Kupfer, besonders bevorzugt 1,40 g bis 1,60 g Kupfer pro Mol umzusetzende Hydroxylgruppe im Polyalkanolamin entspricht.

Das Verhältnis der Masse an MGDA-Na₃ zu CMA-Na₂ im Reaktionsaustrag der oxidativen Dehydrierung kann durch die obigen Bedingungen deutlich zu Gunsten von MGDA-Na₃ beeinflusst und daher die Produktqualität signifikant verbessert werden.

Als Aminopolycarboxylate werden vorliegend Aminocarboxylate mit drei oder vier deprotonierten Carbonsäuregruppen bezeichnet. Aminopolycarboxylate mit drei deprotonierten Carbonsäuregruppen sind insbesondere Salze der Methylglycindiessigsäure sowie der Nitrilotriessigäsure, Aminopolycarboxylate mit vier deprotonierten Carbonsäuregruppen sind insbesondere Salze der Glutaminsäurediessigsäure und Ethylendiamintetraessigsäure. Aufgrund dieser Strukturen können die Aminopolycarboxylate vorteilhaft als Komplexbildner eingesetzt werden.

Das Polyalkanolamin ist vorteilhaft ausgewählt aus der Gruppe der Verbindungen mit der allgemeinen Formel 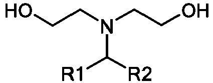 wobei R1 = H oder COOX mit X = Alkali-, Erdalkalimetall oder Wasserstoff ist und R2 = ein Alkyl-, Alkenyl-, Alkinyl-, Aralkyl-, Hydroxyalkyl-, Hydroxyaralkyl-, Alkylencarboxyl-, Alkylensulfonat oder ein Bis(hydroxyethyl)aminoalkylen-Rest ist.

R2 ist bevorzugt ein linearer oder verzweigter Alkylrest mit 1 bis 30 Kohlenstoffatomen, der gegebenenfalls auch Ringe enthalten kann, ein linearer oder verzweigter Alkenylrest mit 2 bis 30 Kohlenstoffatomen, der gegebenenfalls auch Ringe enthalten kann, ein linearer oder verzweigter Hydroxyalkylrest mit 1 bis 30 Kohlenstoffatomen, ein Alkylencarboxylatrest mit 2 bis 30 Kohlenstoffatomen, ein Alkylensulfonatrest mit 1 bis 30 Kohlenstoffatomen oder ein linearer Bis(hydroxyethyl)aminoalkylen-Rest mit 1-10 Kohlenstoffatomen.

Besonders bevorzugt sind Verbindungen die sich aus den Aminosäuren Alanin (R1 = COOX; R2 = CH₃), Glutaminsäure (R1 = COOX; R2 = CH₂-CH₂-COOX) und Serin (R1 = COOX; R2 = CH₂-OH), oder von Ethylendiamin (R1 = H; R2 = CH₂-N(CH₂-CH₂-OH)₂) oder Triethanolamin (R1 = H, R2 = CH₂-OH) ableiten.

Handelt es sich bei den zu Polyalkanolamin um eine chirale Verbindung mit mindestens einem asymmetrischen Kohlenstoffatom, so kann dieses in enantiomerenreiner, skalemischer oder auch racemischer Form eingesetzt werden.

Als Base wird bevorzugt ein Alkali- oder Erdalkalimetallhydroxid, insbesondere Natrium- oder Kaliumhydroxid, eingesetzt. Dies geschieht vorteilhaft als wässrige Lösung, bevorzugt als 50 Gew.-%ige wässrige Lösung.

Der Reaktionsdruck wird vorteilhaft so eingestellt, dass der gebildete Wasserstoff kontinuierlich abgeführt wird. Bevorzugt ist ein Druck von Normaldruck bis 100 bar absolut, besonders bevorzugt von 5-50 bar absolut und ganz besonders bevorzugt von 8-20 bar absolut.

Das Verfahren wird bevorzugt in Batch-Fahrweise druchgeführt.

Ein Verfahren, bei dem das Polyalkanolamin durch Alkoxylierung des zu Grunde liegenden Amins oder der zu Grunde liegenden Aminosäure hergestellt und direkt dehydriert wird, ist besonders bevorzugt. Eine direkte Dehydrierung bedeutet, dass keine auf unterschiedlichen Siedepunkten basierende apparative Abtrennung von Substanzen mit Siedepunkten größer 200°C (bei Normaldruck) zwischen der Alkoxylierung und der oxidativen Dehydrierung erfolgt. Dies ist apparativ einfacher und spart somit eine Operation bei vergleichbar guter Endproduktqualität.

Hierbei kann bevorzugt nach dem in der europäischen Patentanmeldung mit der Anmeldenummer EP 11 162 091.0 beschriebenen Verfahren vorgegangen werden, wonach von einer Aminosäure ausgegangen wird, die in einem ersten Verfahrensschritt mit Ethylenoxid zu einem Zwischenproduktgemisch, enthaltend das entsprechende Dialkanolamin, umgesetzt wird, und danach das Zwischenproduktgemisch in einem zweiten Verfahrensschritt katalytisch unter Einsatz einer Base zum entsprechenden Aminopolycarboxylat umgesetzt wird, wobei die Aminosäure vor der Umsetzung mit Ethylenoxid im ersten Verfahrensschritt einer Teilneutralisation mit 0,70 bis 0,99 Äquivalenten Base pro Säuregruppe zugeführt wird oder wobei im ersten Verfahrensschritt eine bereits mit 0,70 bis 0,99 Äquivalenten Base pro Säuregruppe teilneutralisierte Aminosäure eingesetzt wird.

Der Katalysator, enthaltend 1 bis 90 Gew.-% Kupfer, bezogen auf das Gesamtgewicht desselben, kann z. B. als Pulver oder Formkörper (z. B. Extrudate, Tabletten, etc.), als Vollkontakt oder als Trägerkatalysator eingesetzt werden.

Das Endprodukt des Verfahrens wird gegebenenfalls nach den eingangs beschriebenen, einfachen Nachbehandlungsmaßnahmen, z. B. als Additiv für technische Reinigungsformulierungen für harte Oberflächen aus Metall, Kunststoff, Lack oder Glas, in alkalischen Reinigungsformulierungen für die Getränke- und Nahrungsmittelindustrie, insbesondere für die Flaschenreinigung in der Getränkeindustrie sowie für die Apparatereinigung in Molkereien, in Brauereien, in der Konserven-, der Backwaren-, der Zucker-, der fettverarbeitenden und der fleischverarbeitenden Industrie, in Geschirrreinigungsformulierungen, insbesondere in phosphatfreien Mitteln für das maschinelle Geschirrreinigen in Geschirrspülmaschinen im Haushalt oder in Gewerbebetrieben, z. B. Großküchen oder Restaurants, in Bleichbädern in der Papierindustrie, in photografischen Bleich- und Bleichfixierbädern, bei der Vorbehandlung und Bleichung in der Textilindustrie, in galvanischen Bädern zur Maskierung von verunreinigenden Schwermetallkationen, weiterhin im Bereich der Pflanzenernährung zur Behebung von Schwermetalldefiziten als Kupfer,- Eisen-, Mangan- und/ oder Zinkkomplexe eingesetzt. Der Einsatz ist prinzipiell überall dort vorteilhaft, wo Ausfällungen von Calcium-, Magnesium- oder Schwermetallsalzen technische Verfahren stören und deshalb verhindert werden sollen (Vermeidung von Ablagerungen und Verkrustungen in Kesseln, Rohrleitungen, Sprühdosen oder allgemein an glatten Oberflächen). Außerdem können die Aminopolycarboxylate zur Stabilisierung von Phosphaten in alkalischen Entfettungsbädern und zur Verhinderung der Ausfällung von Kalkseifen verwendet werden, um dadurch das "Anlaufen" von nicht-Eisenoberflächen zu verhindern und die Standzeit von alkalischen Reinigungsbädern zu verlängern. Daneben finden sie Anwendung in Pulver- oder Flüssigwaschmittelformulierungen für die Textilwäsche als Builder und Konservierungsmittel. In Seifen verhindern sie metallkatalysierte, oxidative Zersetzungen wie auch in Pharmazeutika, Kosmetika und Nahrungsmitteln.

Die vorliegende Erfindung wird nachfolgend durch nicht einschränkende Beispiele näher erläutert:

### Herstellung der wässrigen Polyalkanolamin-Ausgangslösung

4,365 kg (49,00 mol) Alanin wurde in 2,623 kg Wasser suspendiert und mit 3,897 kg (49,00 mol) 50,3 Gew.-%iger Natronlauge versetzt. Die resultierende Mischung wurde in einen 20 L-Autoklaven (Material 2.4610) gefüllt und nach entsprechender Inertisierung mit 20 bar Stickstoff beaufschlagt. Anschließend wurde 4,749 kg (107,8 mol) Ethylenoxid bei 40-45 °C innerhalb von 12.5 h zudosiert und noch weitere 3 h bei dieser Temperatur nachgerührt. Nach der Entfernung der nicht umgesetzten Reste an Ethylenoxid wurde der Autoklav entleert. Auf diese Weise wurde 15,634 kg wässriger Reaktionsaustrag als klare, farblose, viskose Lösung erhalten.

### Vergleichsbeispiel 1:

### Oxidative Dehydrierung mit Cu/ZrO₂ als Katalysator

314 g (0,99 mol bezogen auf Alanin) der obigen wässrigen Polyalkanolamin-Ausgangslösung wurden mit 197,9 g (2,39 mol) 50 Gew.-%iger Natronlauge, 32 g Wasser und 3 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 72 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 411 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 72,5% der Theorie bezogen auf eingesetztes Alanin bestimmt. Die Ausbeute an Carboxymethylalanin-Dinatriumsalz (CMA-Na₂) betrug 25,6% der Theorie bezogen auf eingesetztes Alanin. Damit verhält sich die erhaltene Masse an MGDA-Na₃ zur erhaltenen Masse an CMA-Na₂ wie 1 zu 0,25.

### Vergleichsbeispiel 2:

### Oxidative Dehydrierung mit Cu/ZrO₂ als Katalysator

316 g (0,99 mol bezogen auf Alanin) der obigen wässrigen Polyalkanolamin-Ausgangslösung wurden mit 181,6 g (2,27 mol) 50 Gew.-%iger Natronlauge, 32 g Wasser und 30 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 407 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 85,8% der Theorie bezogen auf eingesetztes Alanin bestimmt. Die Ausbeute an Carboxymethylalanin-Dinatriumsalz (CMA-Na₂) betrug 8,6% der Theorie bezogen auf eingesetztes Alanin.

Damit verhält sich die erhaltene Masse an MGDA-Na₃ zur erhaltenen Masse an CMA-Na₂ wie 1 zu 0,07.

Die Raum-Zeit-Ausbeute betrug 7,42 g/l*h.

### Vergleichsbeispiel 3:

### Oxidative Dehydrierung mit Cu/ZrO₂ als Katalysator

316 g (0,99 mol bezogen auf Alanin) der obigen wässrigen Polyalkanolamin-Ausgangslösung wurden mit 181,9 g (2,28 mol) 50 Gew.-%iger Natronlauge, 32 g Wasser und 30 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 180°C aufgeheizt. Diese Temperatur wurde für 24 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 400 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 88,1% der Theorie bezogen auf eingesetztes Alanin bestimmt. Die Ausbeute an Carboxymethylalanin-Dinatriumsalz (CMA-Na₂) betrug 6,8% der Theorie bezogen auf eingesetztes Alanin.

Damit verhält sich die erhaltene Masse an MGDA-Na₃ zur erhaltenen Masse an CMA-Na₂ wie 1 zu 0,05.

Die Raum-Zeit-Ausbeute betrug 5,30 g/l*h.

### Vergleichsbeispiel 4:

### Oxidative Dehydrierung mit Cu/ZrO₂ als Katalysator

316 g (0,99 mol bezogen auf Alanin) der obigen wässrigen Polyalkanolamin-Ausgangslösung wurden mit 181,1 g (2,26 mol) 50 Gew.-%iger Natronlauge, 32 g Wasser und 30 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 170 C aufgeheizt. Diese Temperatur wurde für 60 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 444 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 89,6% der Theorie bezogen auf eingesetztes Alanin bestimmt. Die Ausbeute an Carboxymethylalanin-Dinatriumsalz (CMA-Na₂) betrug 4,3% der Theorie bezogen auf eingesetztes Alanin.

Damit verhält sich die erhaltene Masse an MGDA-Na₃ zur erhaltenen Masse an CMA-Na₂ wie 1 zu 0,03.

Die Raum-Zeit-Ausbeute betrug 2,27 g/l*h.

Die Vergleichsbeispiele 2 bis 4 zeigen, dass eine Durchführung der oxidativen Dehydrierung bei niedrigeren Reaktionstemperaturen eine Erhöhung der Selektivität zu MGDA-Na₃ zur Folge hat. Gleichzeitig sind damit aber deutlich längere Reaktionszeiten verbunden und damit geringere Raum-Zeit-Ausbeuten.

### Beispiel 1 (erfindungsgemäß):

### Oxidative Dehydrierung mit Cu/ZrO₂ als Katalysator

316 g (0,99 mol bezogen auf Alanin) der obigen wässrigen Polyalkanolamin-Ausgangslösung wurden mit 182,3 g (2,28 mol) 50 Gew.-%iger Natronlauge, 32 g Wasser und 30 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 170 °C aufgeheizt. Diese Temperatur wurde für 12 h gehalten, wonach 90 Mol-% des Polyalkanolamins abreagiert waren. Danach wurde innerhalb von 30 Minuten auf 180 °C erwärmt und diese Temperatur anschließend für 4 h gehalten. Danach wurde innerhalb von 30 Minuten auf 190 °C erwärmt und diese Temperatur anschließend für 3 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 419 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 89,4% der Theorie bezogen auf eingesetztes Alanin bestimmt. Die Ausbeute an Carboxymethylalanin-Dinatriumsalz (CMA-Na₂) betrug 5,0% der Theorie bezogen auf eingesetztes Alanin.

Damit verhält sich die erhaltene Masse an MGDA-Na₃ zur erhaltenen Masse an CMA-Na₂ wie 1 zu 0,04.

Die Raum-Zeit-Ausbeute betrug 6,34 g/l*h.

### Beispiel 2 (erfindungsgemäß):

### Oxidative Dehydrierung mit Cu/ZrO₂ als Katalysator

316 g (0,99 mol bezogen auf Alanin) der obigen wässrigen Polyalkanolamin-Ausgangslösung wurden mit 182,0 g (2,28 mol) 50 Gew.-%iger Natronlauge, 32 g Wasser und 30 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 170 °C aufgeheizt. Diese Temperatur wurde für 6 h gehalten, wonach 70 Mol-% des Polyalkanolamins abreagiert waren. Danach wurde innerhalb von 30 Minuten auf 180 °C erwärmt und diese Temperatur anschließend für 4 h gehalten. Danach wurde innerhalb von 30 Minuten auf 190 °C erwärmt und diese Temperatur anschließend für 3 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 394 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 90,0% der Theorie bezogen auf eingesetztes Alanin bestimmt. Die Ausbeute an Carboxymethylalanin-Dinatriumsalz (CMA-Na₂) betrug 5,2% der Theorie bezogen auf eingesetztes Alanin.

Damit verhält sich die erhaltene Masse an MGDA-Na₃ zur erhaltenen Masse an CMA-Na₂ wie 1 zu 0,04.

Die Raum-Zeit-Ausbeute betrug 8,75 g/l*h.

### Beispiel 3 (erfindungsgemäß):

### Oxidative Dehydrierung mit Cu/ZrO2 als Katalysator

316 g (0,99 mol bezogen auf Alanin) der obigen wässrigen Polyalkanolamin-Ausgangslösung wurden mit 182,0 g (2,28 mol) 50 Gew.-%iger Natronlauge, 32 g Wasser und 30 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 170 °C aufgeheizt. Diese Temperatur wurde für 6 h gehalten, wonach 70 Mol-% des Polyalkanolamins abreagiert waren. Danach wurde innerhalb von 30 Minuten auf 180 °C erwärmt und diese Temperatur anschließend für 2 h gehalten. Danach wurde innerhalb von 30 Minuten auf 190 °C erwärmt und diese Temperatur anschließend für 3 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 423 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 88,8% der Theorie bezogen auf eingesetztes Alanin bestimmt. Die Ausbeute an Carboxymethylalanin-Dinatriumsalz (CMA-Na₂) betrug 5,6% der Theorie bezogen auf eingesetztes Alanin.

Damit verhält sich die erhaltene Masse an MGDA-Na₃ zur erhaltenen Masse an CMA-Na₂ wie 1 zu 0,04.

Die Raum-Zeit-Ausbeute betrug 9,84 g/l*h.

Die Beispiele 1-3 zeigen, dass eine erfindungsgemäße Fahrweise in kürzeren Reaktionszeiten zu gleichen Ausbeuten an Wertprodukt führt wie eine konstante Fahrweise bei niedriger Temperatur und entsprechend längerer Reaktionszeit.

### Vergleichsbeispiel 5:

### Oxidative Dehydrierung mit Cu/ZrO₂ als Katalysator

316 g (0,99 mol bezogen auf Alanin) der obigen wässrigen Polyalkanolamin-Ausgangslösung wurden mit 182,4 g (2,28 mol) 50 Gew.-%iger Natronlauge, 182 g Wasser und 10 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 256 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 49,4% der Theorie bezogen auf eingesetztes Alanin bestimmt. Die Ausbeute an Carboxymethylalanin-Dinatriumsalz (CMA-Na₂) betrug 12,9% der Theorie bezogen auf eingesetztes Alanin.

Damit verhält sich die erhaltene Masse an MGDA-Na₃ zur erhaltenen Masse an CMA-Na₂ wie 1 zu 0,18.

### Vergleichsbeispiel 6:

### Oxidative Dehydrierung mit Cu/ZrO₂ als Katalysator

316 g (0,99 mol bezogen auf Alanin) der obigen wässrigen Polyalkanolamin-Ausgangslösung wurden mit 181,9 g (2,28 mol) 50 Gew.-%iger Natronlauge, 32 g Wasser und 10 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 425 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 76,2% der Theorie bezogen auf eingesetztes Alanin bestimmt. Die Ausbeute an Carboxymethylalanin-Dinatriumsalz (CMA-Na₂) betrug 16,0% der Theorie bezogen auf eingesetztes Alanin.

Damit verhält sich die erhaltene Masse an MGDA-Na₃ zur erhaltenen Masse an CMA-Na₂ wie 1 zu 0,15.

### Vergleichsbeispiel 7:

### Oxidative Dehydrierung mit Cu/ZrO₂ als Katalysator

253 g (0,98 mol bezogen auf Alanin) der durch Destillation aufkonzentrierten obigen wässrigen Polyalkanolamin-Ausgangslösung wurden mit 182,3 g (2,28 mol) 50 Gew.-%iger Natronlauge, 37 g Wasser und 10 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 431 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 81,8% der Theorie bezogen auf eingesetztes Alanin bestimmt. Die Ausbeute an Carboxymethylalanin-Dinatriumsalz (CMA-Na₂) betrug 13,9% der Theorie bezogen auf eingesetztes Alanin.

Damit verhält sich die erhaltene Masse an MGDA-Na₃ zur erhaltenen Masse an CMA-Na₂ wie 1 zu 0,12.

### Vergleichsbeispiel 8:

### Oxidative Dehydrierung mit Cu/ZrO₂ als Katalysator

274 g (0,98 mol bezogen auf Alanin) der durch Destillation aufkonzentrierten obigen wässrigen Polyalkanolamin-Ausgangslösung wurden mit 182,6 g (2,28 mol) 50 Gew.-%iger Natronlauge und 10 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 471 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 70,4% der Theorie bezogen auf eingesetztes Alanin bestimmt. Die Ausbeute an Carboxymethylalanin-Dinatriumsalz (CMA-Na₂) betrug 7,8% der Theorie bezogen auf eingesetztes Alanin.

Damit verhält sich die erhaltene Masse an MGDA-Na₃ zur erhaltenen Masse an CMA-Na₂ wie 1 zu 0,08.

Die Vergleichsbeispiele 5 bis 8 zeigen, dass für die Selektivität der oxidativen Dehydrierung der Wassergehalt in der Reaktionsmischung eine wichtige Rolle spielt, jedoch lässt sich allein über die Einstellung des Wassergehaltes noch keine ausreichende Selektivität erreichen.

### Vergleichsbeispiel 9:

### Oxidative Dehydrierung mit Cu/ZrO₂ als Katalysator

316 g (0,99 mol bezogen auf Alanin) der obigen wässrigen Polyalkanolamin-Ausgangslösung wurden mit 181,7 g (2,27 mol) 50 Gew.-%iger Natronlauge, 32 g Wasser und 10 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 404 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 77,2% der Theorie bezogen auf eingesetztes Alanin bestimmt. Die Ausbeute an Carboxymethylalanin-Dinatriumsalz (CMA-Na₂) betrug 18,2% der Theorie bezogen auf eingesetztes Alanin.

Damit verhält sich die erhaltene Masse an MGDA-Na₃ zur erhaltenen Masse an CMA-Na₂ wie 1 zu 0,17.

### Vergleichsbeispiel 10:

### Oxidative Dehydrierung mit Cu/ZrO₂ als Katalysator

316 g (0,99 mol bezogen auf Alanin) der obigen wässrigen Polyalkanolamin-Ausgangslösung wurden mit 182,1 g (2,28 mol) 50 Gew.-%iger Natronlauge, 32 g Wasser und 20 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 411 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 77,8% der Theorie bezogen auf eingesetztes Alanin bestimmt. Die Ausbeute an Carboxymethylalanin-Dinatriumsalz (CMA-Na₂) betrug 11,3% der Theorie bezogen auf eingesetztes Alanin.

Damit verhält sich die erhaltene Masse an MGDA-Na₃ zur erhaltenen Masse an CMA-Na₂ wie 1 zu 0,10.

### Vergleichsbeispiel 11:

### Oxidative Dehydrierung mit Cu/ZrO₂ als Katalysator

316 g (0,99 mol bezogen auf Alanin) der obigen wässrigen Polyalkanolamin-Ausgangslösung wurden mit 181,6 g (2,27 mol) 50 Gew.-%iger Natronlauge, 32 g Wasser und 30 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 407 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 85,8% der Theorie bezogen auf eingesetztes Alanin bestimmt. Die Ausbeute an Carboxymethylalanin-Dinatriumsalz (CMA-Na₂) betrug 8,6% der Theorie bezogen auf eingesetztes Alanin.

Damit verhält sich die erhaltene Masse an MGDA-Na₃ zur erhaltenen Masse an CMA-Na₂ wie 1 zu 0,07.

### Vergleichsbeispiel 12:

### Oxidative Dehydrierung mit Cu/ZrO₂ als Katalysator

316 g (0,99 mol bezogen auf Alanin) der obigen wässrigen Polyalkanolamin-Ausgangslösung wurden mit 181,8 g (2,27 mol) 50 Gew.-%iger Natronlauge, 32 g Wasser und 40 g Cu/ZrO₂ (Nachstellung Patent DE 3505208) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 400 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 89,5% der Theorie bezogen auf eingesetztes Alanin bestimmt. Die Ausbeute an Carboxymethylalanin-Dinatriumsalz (CMA-Na₂) betrug 7,5% der Theorie bezogen auf eingesetztes Alanin.

Damit verhält sich die erhaltene Masse an MGDA-Na₃ zur erhaltenen Masse an CMA-Na₂ wie 1 zu 0,06.

Die Vergleichsbeispiele 9 bis 12 zeigen, dass steigende eingesetzte Katalysatormengen nicht nur die Geschwindigkeit sondern auch die Selektivität der oxidativen Dehydrierung erhöhen. Jedoch lässt sich allein über die Einstellung der Katalysatormenge noch keine ausreichende Selektivität erreichen.

## Patentansprüche

1. Verfahren zur Herstellung von Aminopolycarboxylaten ausgehend von den entsprechenden Polyalkanolaminen durch oxidative Dehydrierung in Gegenwart eines Katalysators enthaltend 1 bis 90 Gew.-% Kupfer, bezogen auf das Gesamtgewicht des Katalysators, unter Einsatz einer Base, **dadurch gekennzeichnet, dass** zunächst eine Teilumsetzung des Polyalkanolamins zu einem das Aminopolycarboxylat enthaltenden Reaktionsgemisch bei einer Temperatur im Bereich von 140 bis 180 °C durchgeführt wird, bis mindestens 10 bis 90 Mol-% des Polyalkanolamins abreagiert sind, und dass anschließend die Umsetzung bei erhöhter Temperatur fortgeführt wird.

2. Verfahren nach Anspruch 1, **dadurch gekennzeichnet, dass** das Alkanolamin ausgewählt ist aus der Gruppe der Polyalkanolamine der Formel: 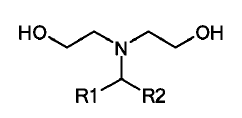 wobei R1 = H oder COOX mit X = Alkali-, Erdalkalimetall oder Wasserstoff ist und R2 = ein Alkyl-, Alkenyl-, Alkinyl-, Aralkyl-, Hydroxyalkyl-, Hydroxyaralkyl-, Alkylencarboxyl-, Alkylensulfonat- oder ein Bis(hydroxyethyl)aminoalkylen-Rest ist.

3. Verfahren nach Anspruch 2, **dadurch gekennzeichnet, dass** R1 = H und R2 = -CH₂OH, oder R1 = COOX und R2 = CH₃, oder R1 = COOX und R2 = CH₂-CH₂-COOX, oder R1 = H und R2 = CH₂-N(C₂H₄OH)₂, ist.

4. Verfahren nach einem der Ansprüche 1 bis 3, **dadurch gekennzeichnet, dass** der Temperaturbereich, in dem die Teilumsetzung durchgeführt wird, 150 bis 175 °C, bevorzugt 165 bis 175 °C, beträgt.

5. Verfahren nach einem der Ansprüche 1 bis 4, **dadurch gekennzeichnet, dass** die Menge des abreagierten Polyalkanolamins vor der Erhöhung der Reaktionstemperatur 30 bis 90 Mol-%, bevorzugt 50 bis 80 Mol-%, beträgt.

6. Verfahren nach einem der Ansprüche 1 bis 5, **dadurch gekennzeichnet, dass** die Temperatur nach der Teilumsetzung direkt oder schrittweise auf 180 bis 200 °C, bevorzugt auf 185 bis 195 °C, erhöht wird.

7. Verfahren nach einem der Ansprüche 1 bis 6, **dadurch gekennzeichnet, dass** die katalytische oxidative Dehydrierung in Gegenwart von Wasser als Lösungsmittel durchgeführt wird.

8. Verfahren nach Anspruch 7, **dadurch gekennzeichnet, dass** die Konzentration des Wassers im Reaktionsgemisch 30 bis 80 Gew.-%, bevorzugt 35 bis 60 Gew.-%, besonders bevorzugt 40 bis 55 Gew.-%, beträgt.

9. Verfahren nach einem der Ansprüche 1 bis 7, **dadurch gekennzeichnet, dass** die eingesetzte Katalysatormenge so bemessen wird, dass sie 0,40 g bis 2,00 g Kupfer, bevorzugt 1,00 g bis 1,70 g Kupfer, besonders bevorzugt 1,40 g bis 1,60 g Kupfer pro Mol umzusetzender Hydroxylgruppe im Polyalkanolamin entspricht.

10. Verfahren nach einem der Ansprüche 1 bis 9, **dadurch gekennzeichnet, dass** als Polyalkanolamin ein Zwischenproduktgemisch, enthaltend ein Dialkanolamin, eingesetzt wird, das durch Ethoxylierung einer Aminosäure erhalten wird, wobei die Aminosäure vor der Umsetzung mit Ethylenoxid einer Teilneutralisation mit 0,70 bis 0,99 Äquivalenten Base pro Säuregruppe zugeführt wird oder dass der Umsetzung mit Ethylenoxid eine bereits mit 0,70 bis 0,99 Äquivalenten Base pro Säuregruppe teilneutralisierte Aminosäure zugeführt wird.

## Claims

1. A process for preparing aminopolycarboxylates proceeding from the corresponding polyalkanolamines by oxidative dehydrogenation in the presence of a catalyst comprising 1 to 90% by weight of copper, based on the total weight of the catalyst, using a base, which comprises first performing a partial conversion of the polyalkanolamine to a reaction mixture comprising the aminopolycarboxylate at a temperature in the range from 140 to 180°C until at least 10 to 90 mol% of the polyalkanolamine has been reacted, and then continuing the conversion at elevated temperature.

2. The process according to claim 1, wherein the alkanolamine is selected from the group of the polyalkanolamines of the formula: 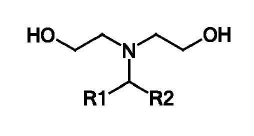 where R1 = H or COOX where X = alkali metal, alkaline earth metal or hydrogen, and R2 = an alkyl, alkenyl, alkynyl, aralkyl, hydroxyalkyl, hydroxyaralkyl, alkylene carboxyl, alkylene sulfonate or a bis- (hydroxyethyl)aminoalkylene radical.

3. The process according to claim 2, wherein R1 = H and R2 = -CH₂OH, or R1 = COOX and R2 = CH₃, or R1 = COOX and R2 = CH₂-CH₂-COOX, or R1 = H and R2 = CH₂-N(C₂H₄OH)₂.

4. The process according to any of claims 1 to 3, wherein the temperature range within which the partial conversion is performed is 150 to 175°C, preferably 165 to 175°C.

5. The process according to any of claims 1 to 4, wherein the amount of the reacted polyalkanolamine before the increase in the reaction temperature is 30 to 90 mol%, preferably 50 to 80 mol%.

6. The process according to any of claims 1 to 5, wherein the temperature is increased after the partial conversion directly or stepwise to from 180 to 200°C, preferably to from 185 to 195°C.

7. The process according to any of claims 1 to 6, wherein the catalytic oxidative dehydrogenation is performed in the presence of water as a solvent.

8. The process according to claim 7, wherein the concentration of the water in the reaction mixture is 30 to 80% by weight, preferably 35 to 60% by weight, more preferably 40 to 55% by weight.

9. The process according to any of claims 1 to 7, wherein the amount of catalyst used is such that it corresponds to 0.40 g to 2.00 g of copper, preferably 1.00 g to 1.70 g of copper and more preferably 1.40 g to 1.60 g of copper per mole of hydroxyl group to be converted in the polyalkanolamine.

10. The process according to any of claims 1 to 9, wherein the polyalkanolamine used is an intermediate mixture comprising a dialkanolamine which is obtained by ethoxylating an amino acid, the amino acid being supplied to a partial neutralization with 0.70 to 0.99 equivalent of base per acid group before the reaction with ethylene oxide, or an amino acid already partly neutralized with 0.70 to 0.99 equivalent of base per acid group is supplied to the reaction with ethylene oxide.

## Revendications

1. Procédé pour la préparation d'aminopolycarboxylates, partant des polyalcanolamines correspondantes par déshydrogénation oxydante en présence d'un catalyseur contenant 1 à 90% en poids de cuivre, par rapport au poids total du catalyseur, avec utilisation d'une base, **caractérisé en ce qu'**on réalise d'abord une conversion partielle de la polyalcanolamine en un mélange réactionnel contenant l'aminopolycarboxylate à une température dans la plage de 140 à 180°C jusqu'à ce qu'au moins 10 à 90% en mole de la polyalcanolamine aient réagi et **en ce que** qu'on continue ensuite la conversion à une température augmentée.

2. Procédé selon la revendication 1, **caractérisé en ce que** l'alcanolamine est choisie dans le groupe des polyalcanolamines de formule : 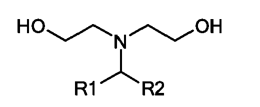 R1 = H ou COOX avec X = un métal alcalin, un métal alcalino-terreux ou hydrogène et R2 = un radical alkyle, alcényle, alcynyle, aralkyle, hydroxyalkyle, hydroxyaralkyle, alkylènecarboxyle, alkylènesulfonate ou bis(hydroxyéthyl)aminoalkylène.

3. Procédé selon la revendication 2, **caractérisé en ce que** R1 = H et R2 = -CH₂OH, ou R1 = COOX et R2 = CH₃, ou R1 = COOX et R2 = CH₂-CH₂-COOX, ou R1 = H et R2 = CH₂-N(C₂H₄OH)₂.

4. Procédé selon l'une quelconque des revendications 1 à 3, **caractérisé en ce que** la plage de température dans laquelle la conversion partielle est réalisée est de 150 à 175°C, de préférence de 165 à 175°C.

5. Procédé selon l'une quelconque des revendications 1 à 4, **caractérisé en ce que** la quantité de la polyalcanolamine qui a réagi avant l'augmentation de la température de réaction est de 30 à 90% en mole, de préférence de 50 à 80% en mole.

6. Procédé selon l'une quelconque des revendications 1 à 5, **caractérisé en ce qu'**après la conversion partielle, la température est augmentée directement ou par étapes à 180 jusqu'à 200°C, de préférence à 185 jusqu'à 195°C.

7. Procédé selon l'une quelconque des revendications 1 à 6, **caractérisé en ce que** la déshydrogénation oxydante catalytique est réalisée en présence d'eau comme solvant.

8. Procédé selon la revendication 7, **caractérisé en ce que** la concentration de l'eau dans le mélange réactionnel est de 30 à 80% en poids, de préférence de 35 à 60% en poids, de manière particulièrement préférée de 40 à 55% en poids.

9. Procédé selon l'une quelconque des revendications 1 à 7, **caractérisé en ce que** la quantité catalytique utilisée est dimensionnée de manière telle qu'elle correspond à 0,40 g jusqu'à 2,00 g de cuivre, de préférence à 1,00 g jusqu'à 1,70 g de cuivre, de manière particulièrement préférée à 1,40 g jusqu'à 1,60 g de cuivre, par mole de groupe hydroxyle à transformer dans la polyalcanolamine.

10. Procédé selon l'une quelconque des revendications 1 à 9, **caractérisé en ce qu'**on utilise, comme polyalcanolamine, un mélange de produits intermédiaires, contenant une dialcanolamine qui est obtenue par éthoxylation d'un aminoacide, l'aminoacide étant acheminé, avant la transformation avec de l'oxyde d'éthylène, vers une neutralisation partielle avec 0,70 à 0,99 équivalent de base par groupe acide ou **en ce qu'**un aminoacide déjà partiellement neutralisé par 0,70 à 0,99 équivalent de base par groupe acide est acheminé vers la transformation avec l'oxyde d'éthylène.
